## Europäisches Patentamt
## European Patent Office
## Office européen des brevets

(19)

(11) Numéro de publication: **0 182 722**
**B1**

(12) **FASCICULE DE BREVET EUROPEEN**

(45) Date de publication du fascicule du brevet: **16.08.90**

(51) Int. Cl.⁵: **A 61 M 16/00**

(21) Numéro de dépôt: **85402255.5**

(22) Date de dépôt: **20.11.85**

(54) **Appareil de ventilation artificielle pourvu d'un dispositif d'assistance inspiratoire volumétrique.**

(30) Priorité: **20.11.84 FR 8417653**

(43) Date de publication de la demande:
**28.05.86 Bulletin 86/22**

(45) Mention de la délivrance du brevet:
**16.08.90 Bulletin 90/33**

(84) Etats contractants désignés:
**CH DE GB IT LI SE**

(56) Documents cités:
**FR-A-2 353 305**
**GB-A-2 054 387**

(73) Titulaire: **B O C S.A. Société anonyme dite**
**Z.I. de Coignières-Maurepas Boulevard des**
**Arpents B.P. No. 186**
**F-78313 Maurepas-Cedex (FR)**

(72) Inventeur: **Zalkin, Daniel**
**7, rue du Tournebride**
**F-78120 Rambouillet (FR)**

(74) Mandataire: **Bruder, Michel**
**10 rue de la Pépinière**
**F-75008 Paris (FR)**

Courier Press, Leamington Spa, England.

EP 0 182 722 B1

**Description**

La présente invention concerne un appareil de ventilation artificielle pour l'assistance inspiratoire volumétrique d'un patient, tel que defini dans le préambule de la revendication 1.

Les appareils de ventilation artificielle modernes de ce type, tels que par exemple celui décrit dans le brevet GB—2054387, utilisent des modes ventilatoires dans lesquels un patient respire d'une manière intermittente ou continue de façon spontanée. Avec de tels appareils, lorsque la patient inspire spontanément dans le circuit de l'appareil, un dispositif détecte la baisse de pression apparaissant au début de l'inspiration, ou "appel", et provoque la mise en pression du circuit à un niveau supérieur de quelques millibars au niveau initial, afin d'aider le transfert du gaz insufflé vers les poumons. Ce niveau de pression est prédéterminé par la prescripteur de la ventilation. Lorsque le patient a reçu un volume de gaz tel que l'élévation de la pression intrapulmonaire soit égale à l'élévation de la pression du circuit, la résistance qu'il oppose alors au débit de gaz provoque une interruption de la phase inspiratoire. On assure ainsi au patient, par une commande de débit, un débit de gaz d'inspiration correspondant à une pression d'équilibre ou pression positive de fin d'expiration PEEP en détectant la baisse de pression du flux de gaz vers le patient du fait de l'inspiration et en augmentant le débit de ce flux de gaz proportionnellement à la baisse de pression détectée par rapport à cette pression d'équilibre PEEP.

L'appareil mettant en oeuvre un tel procédé comprend une source d'alimentation en gaz sous pression, une canalisation d'inspiration reliée au patient, une valve demande branchée entre la source d'alimentation et la canalisation d'inspiration, cette valve demande constituant des moyens de commande du débit ge gaz distribué vers le patient du fait qu'elle comprend une chambre de référence reliée d'une part à une canalisation d'expiration reliée au patient et d'autre part à la sortie d'un venturi relié par son entrée à une canalisation d'alimentation la reliant à la source d'alimentation en gaz afin de commander la valve demande pour qu'elle envoie vers le patient le gaz à la pression d'équilibre PEEP.

L'intérêt d'une tel procédé est évident pour un patient ayant des performances inspiratoires insuffisantes, puisqu'il est démontré que, sur le plan hémodynamique, une ventilation spontanée prédéterminée est préférable à une ventilation contrôlée en pression positive intermittente, puisque la pression intrapulmonaire obtenue dans le premier cas est notablement plus basse que dans le second. Toutefois cette technique présente un inconvénient important à savoir que l'assistance inspiratoire induit, pour le patient, un confort qui ne l'incite pas à l'effort. Une telle situation rend, par conséquent, le sevrage difficile et conduit à la nécessité d'effectuer des réglages fréquents du seuil de pression d'assistance.

La présente invention vise à remédier à ces inconvénients en proposant un appareil conforme à la revendication 1.

On propose donc par cette invention un dispositif d'assistance inspiratoire volumétrique de conception particulièrement simple et qui permet de réaliser une assistance nécessaire et suffisante pour obtenir des volumes courants spontanés égaux ou supérieurs à un seuil prédéterminé par le prescripteur. L'appareil est conçu de telle façon que le patient interrompt, si nécessaire, le cycle resporatoire, mais sans provoquer des variations importantes de pression dans son circuit de ventilation artificielle d'assistance.

On décrira ci-après, à titre d'exemple non limitatif, une forme d'exécution de la présente invention, en référence au dessin annexé sur lequel:

La figure 1 est un schéma synoptique d'un appareil de ventilation artificielle pourvu d'un dispositif d'assistance inspiratoire volumétrique suivant l'invention.

La figure 2 est un diagramme illustrant la variation de la pression et du débit au cours des phases d'inspiration et d'expiration d'un cycle respiratoire complet, en fonction du temps.

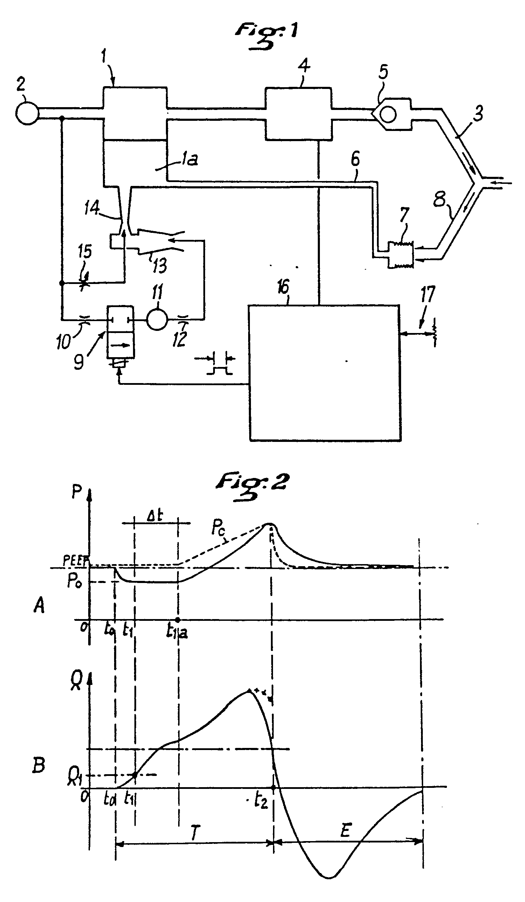

L'appareil de ventilation artificielle suivant l'invention ainsi qu'il est représenté sur la figure 1, comporte une valve demande 1 alimentée en gaz respirable sous pression à partir d'une source de gaz 2. Une telle valve demande est un organe bien connu en soi qui est en fait un générateur de débit de gaz dont la commande est constituée par un système de détection de baisse de pression par rapport à une pression d'équilibre PEEP (pression positive de fin d'expiration), par exemple du fait de l'inspiration du patient, et dont le débit de sortie est proportionnel a cette baisse de pression. La sortie de cette valve demande 1 communique avec une canalisation d'inspiration 3, reliée à un patient devant bénéficier de l'assistance inspiratoire, et ce par l'intermédiaire d'un capteur de débit d'inspiration 4 et d'un clapet anti-retour 5. La valve demande 1 comporte par ailleurs une chambre de référence 1a qui est reliée, par une conduite de prise de pression 6, à l'intérieur d'un soufflet d'une soupape d'expiration 7 branchée sur une canalisation d'expiration 8 et reliée au patient. Ce circuit constitue une première commande de la valve demande 1, provoquant un débit de sortie de la valve demande 1 proportionnel à la baisse de la pression dans le circuit par rapport à la pression d'équilibre PEEP. La valeur de cette pression PEEP est réglable par des moyens décrits plus loin.

La valve demande 1 comporte également une seconde commande de débit qui est constituée par un système d'auqmentation de pression dans la canalisation 3 par rapport à la pression d'équilibre PEEP mentionnée précédemment. Cette seconde commande de pression est constituée par un générateur de pression croissante 9—13 comprenant une électrovanne 9 reliée d'une part à la source d'alimentation en gaz 2, par l'intermédiaire d'un premier étranglement 10, et d'autre part à la chambre de référence 1a de la valve commande 1 par l'intermédiaire d'une capacité 11, d'un deuxième étranglement 12 et d'un second venturi 13 d'assistance. Ce second venturi 13 débouche lui-même dans l'extrémité amont d'un premier venturi 14 produisant la pression d'équilibre PEEP. La valeur de cette pression d'équilibre PEEP peut être réglée au moyen d'un étranglement variable 15 branché sur une canalisation d'alimentation du venturi 14, laquelle est reliée à la source d'alimentation en gaz 2.

L'électrovanne 9 est connectée à un calculateur 16 qui est relié d'une part au capteur de débit d'inspiration 4 et d'autre part à un organe 17, tel qu'un potentiomètre, de réglage du volume courant spontané minimal que reçoit le patient par la canalisation d'inspiration 3.

Avec l'appareil suivant l'invention, le patient sollicite tout d'abord la première commande de la valve demande 1 pour obtenir le débit nécessaire à la reconstitution de la pression PEEP et ensuite la seconde commande c'est-à-dire le dispositif d'assistance prévu suivant l'invention, constituant la commande d'augmentation de pression, pour achever, par un débit complémentaire, le transfert d'un débit de gaz nécessaire à l'obtention du volume prédéterminé par le prescripteur de l'assistance respiratoire pour constituer un seuil de volume courant minimal le gaz envoyé au patient. Le capteur de débit 4 fournit au calculateur 16 un signal correspondant au volume de gaz délivré au patient. Le calculateur 16 effectue alors le calcul en temps réel du volume à délivrer et il commande la mise en service de l'assistance inspiratoire notamment de la seconde commande. L'organe ou potentiomètre 17 fixe au calculateur 16 le seuil minimal de volume courant. Le générateur de pression croissante constitué par l'ensemble des organes 9—13 crée, sur l'ordre du calculateur 16, une augmentation progressive de pression, par rapport à la pression d'équilibre PEEP, appliquée dans la chambre de référence 1a de la valve demande 1 pour réaliser le débit effectif à l'inspiration envoyé au patient.

Le fonctionnement détaillé de l'appareil qui vient d'être décrit est le suivant:

Au début d'un cycle d'inspiration T (voir le diagramme de la figure 2), le patient provoque, à l'instant $t_0$ correspondant au début de l'inspiration, un appel dans le circuit 3, 4, 5 de liaison à la valve demande 1, appel qui se traduit par une baisse de la pression P (diagramme A du haut de la figure) par rapport à la pression d'équilibre PEEP. Cette baisse de pression jusqu'à la valeur Po commande l'ouverture de la valve demande 1 qui laisse alors passer, vers le patient, un débit Q (diagramme B du bas de la figure) croissant à partir de Q à l'instant $t_0$. La perception de ce débit par le capteur 4 initialise le cycle inspiratoire pour le calculateur 16. Celui-ci donne l'ordre au générateur de pression 9—13 de fonctionner et de créer la seconde commande, progressive, de la valve demande 1, tendant à augmenter progressivement le débit imposé au patient. Cet ordre se traduit par l'ouverture de l'électrovanne 9. Le second venturi 13 d'assistance est alors alimenté à travers cette électrovanne 9, l'étranglement 10, la capacité 11 et l'étranglement 12.

L'ouverture de la vanne 9, donc le début de la mise en service de l'assistance respiratoire a lieu à l'instant $t_1$ où est détecté l'envoi vers le patient d'un débit d'inspiration Q1 correspondant au seuil d'ouverture du dispositif d'assistance (9—13).

Par ailleurs, en temps réel, le calculateur 16 intègre le débit Q allant au patient et qui est mesuré par le capteur 4. Lorsque le volume résultant de cette intégration atteint la valeur du seuil prédéterminé, qui est réglée au moyen de l'organe 17 de réglage du volume courant spontané minimal, le calculateur 16 annule l'ordre d'excitation de l'électrovanne 9, de manière à provoquer la fermeture de celle-ci, ce qui interrompt la commande du générateur de pression (9—13) à l'instant $t_2$. De ce fait la seconde commande de la valve demande 1 est annulée, ce qui entraîne la suppression de l'assistance inspiratoire et la baisse de la pression dans le circuit 1—4—5—3 vers la valeur PEEP.

Un temps maximal d'application de l'assistance respiratoire, c'est-à-dire de la seconde commande de la valve demande 1, est défini par le calculateur 16 en fonction du seuil de volume courant minimal prédéterminé par le prescripteur, c'est-à-dire du réglage de l'organe 17, tenant compte des débits admissibles par le patient et du résultat obtenu lors de sont premier cycle respiratoire, inspiration-expiration.

Après expiration du patient (phase E du cycle) un nouveau cycle T—E respiratoire commence et une nouvelle inspiration est effectuée (phase T du cycle). Au cours de ce deuxième cycle le calculateur 16 intervient pour retarder l'application de l'assistance respiratoire. Ce retard Δt correspond à une fraction du temps d'assistance calculé à partir de l'instant t1 de commencement de l'assistance. Comme on peut le voir sur la figure 2 la pression de commande Pc de la valve demande 1 indiquée par la courbe en tirets sur le diagramme A de la figure 2, commence à croître à partir d'un instant t1a décalé d'un intervalle de temps Δt par rapport à l'instant t1. De ce fait la mise en service du générateur de pression 9—13, c'est-à-dire l'ouverture de l'électrovanne 9 sont retardées de l'intervalle de temps Δt par rapport à l'instant t1 de détection de l'inspiration. A la fin du deuxième cycle respiratoire T—E le calculateur 16 vérifie que le volume inspiré par le patient est encore égal ou supérieur au seuil prédéterminé par l'organe de réglage 17. Dans ce cas lors du troisième cycle respiratoire, l'application de l'assistance respiratoire, c'est-à-dire la mise en service du générateur de pression 9—13 est retardée d'une valeur Δt' qui est supérieure au retard Δt introduit lors du deuxième cycle précédent. Dans le cas contraire, si le volume inspiré est inférieur au seuil prédéterminé, le retard Δt est alors diminué au cours du troisième cycle. Ainsi de cycle en cycle le retard est modifié jusqu'à ce que soit atteinte la valeur Δt qui assure l'assistance inspiratoire minimale permettant d'obtenir l'insufflation du volume courant prédéterminé.

**Revendications**

1. Appareil de ventilation artificielle pour l'assistance inspiratoire volumétrique d'un patient, comprenant une source (2) d'alimentation en gaz sous pression, une canalisation d'inspiration (3) reliée au patient et une valve de demande (1), branchée entre la source d'alimentation (2) et la canalisation d'inspiration (3), cette valve de demande (1) constituant des moyens de commande de débit de gaz distribué vers le patient du fait qu'elle comprend une chambre de référence (1a) reliée d'une part à une canalisation d'expiration (8) reliée au patient et, d'autre part à la sortie d'un venturi (14), relié par son entrée à une canalisation d'alimentation, qui est relié à la source d'alimentation en gaz (2), ledit venturi (14) produisant une pression d'équilibre prédéterminée PEEP dans la chambre de référence (1a) afin de commander la valve de demande (1) pour qu'elle envoie le gaz, vers le patient si la pression dans la canalisation d'inspiration baisse par rapport à la pression d'équilibre PEEP, comprenant un générateur de pression croissante (9—13), par transfert d'un débit complémentaire de gaz, relié par son entrée à la source d'alimentation en gaz (2) et débouchant dans l'extrémité amont du venturi (14), ce générateur étant commandé par un calculateur (16) relié à un capteur (4) de débit de gaz fournissant au calculateur (16) un signal représentant le volume de gaz délivré au patient, de manière à interrompre automatiquement l'assistance inspiratoire après qu'un volume de gaz prédéterminé a été délivré au patient au cours d'un cycle respiratoire spontané, caractérisé en ce que le générateur de pression croissante (9—13) comprend un second venturi (13) relié, par son extrémité aval, à l'extrémité amont du premier venturi (14) et par son extrémité amont à une électrovanne (9) branchée entre la source d'alimentation en gaz (2), par l'intermédiaire d'un étranglement (10), et le second venturi (13) par l'intermédiaire d'une capacité (11) et d'une autre étranglement (12), l'électrovanne (9) étant reliée à la sortie du calculateur (16).

2. Appareil suivant la revendication 1 caractérisé en ce que le calculateur (16) est pourvu d'un organe (17), tel qu'un potentiomètre, prédéterminant le seuil du volume courant minimal de gaz décidé par le prescripteur de l'assistance respiratoire.

**Patentansprüche**

1. Gerät zur künstlichen Beatmung mit volumetrischer Inspirationsunterstützung des Patienten, mit einer Quelle (2) zur Versorgung mit Druckgas, mit einer an den Patienten angeschlossenen Inspirationsleitung (3) und mit einem Zuführungsventil (1), welches zwischen die Versorgungsquelle (2) und die Inspirationsleitung (3) geschaltet ist und eine Vorrichtung zur Steuerung der dem Patienten zugeführten Gasmenge bildet, indem es eine Referenzkammer (1a) enthält, die einerseits mit einer an den Patienten angeschlossenen Expirationsleitung (8) und andererseits mit dem Auslaß eines Venturirohres (14) verbunden ist, welches mit seinem Eingang an eine mit der Gasversorgungsquelle (2) verbundene Versorgungsleitung angeschlossen ist und einen vorgegebenen Gleichgewichtsdruck PEEP in der Referenzkammer (1a) erzeugt, um das Zuführungsventil (1) so zu steuern, daß es Gas an den Patienten liefert, wenn der Druck in der Inspirationsleitung bezüglich des Gleichgewichtsdruckes PEEP absinkt, mit einem Generator (9—13) für eine Druckerhöhung durch Übertragung einer komplementären Gasmenge, der mit seinem Einlaß mit der Gasversorgungsquelle (2) verbunden ist, mit seinem Ende stromaufwärts vom Venturirohr (14) mündet und durch einen Rechner (16) gesteuert ist, welcher mit einem Gasmengenaufnehmer verbunden ist, der an den Rechner (16) ein Signal entsprechend dem Volumen des dem Patienten zugeführten Gases liefert, derart daß die Inspirationsunterstützung automatisch unterbrochen wird, nachdem ein vorgegebenes Gasvolumen im Zuge eines spontanen Atemzyklus an den Patienten abgegeben worden ist, dadurch gekennzeichnet, daß der Generator (9—13) für den Druckanstieg ein zweites Venturirohr (13) enthält, welches mit seinem stromabwärtigen Ende mit dem stromaufwärtigen Ende des ersten Venturirohres (14) und mit seinem stromaufwärtigen Ende mit einem Elektroventil (9) verbunden ist, das zwischen die Gasversorgungsquelle (2) über eine Drossel (10) und das zweite Venturirohr (13) über eine Kapazität (11) sowie eine andere Drossel (22) geschaltet und an den Ausgang des Rechners (16) angeschlossen ist.

2. Gerät nach Anspruch 1, dadurch gekennzeichnet, daß der Rechner (16) mit einer Vorrichtung (17), wie einem Potentiometer, versehen ist, welche die Schwelle des minimalen Gasvolumenstromes bestimmt, welcher von dem die Atemunterstützung Verordnenden festgelegt wurde.

**Claims**

1. An apparatus for artificial ventilation for assisting the volumetric inspiration of a patient comprising a source (2) of supply of pressurized gas, an inspiration tube (3) connected to the patient and a demand valve (1) connected between the source of supply (2) and the inspiration tube (3), this demand valve (1) constituting means for controlling the flow rate of gas distributed towards the patient as it comprises a reference chamber (1a) connected on the one hand to an expiration tube (8) connected to the patient and on the other hand to the output of a Venturi tube (14) connected by its inlet to a supply tube which is connected to the source (2) of supply of gas said Venturi tube (14) generating a predetermined balance pressure PEEP in the reference chamber (1a) in order to control the demand valve (1) so that it sends the gas to the patient if the pressure in the inspiration tube decreases with respect to the balanced pressure PEEP, comprising a generator of increasing pressure (9—13) by transfer of a complementary flow of gas, connected by its inlet to the source (2) of gas supply and opening out in the upstream end of the Venturi tube (14), this generator being controlled by a computer (16) connected to a sensor (4) detecting the flowrate of gas sending to the computer (16) a signal indicating the volume of gas supplied to the patient in order to interrupt the assisted respiration after the supply of a predetermined volume of gas to the patient during a spontaneous respiratory cycle characterized in that the increasing pressure generator (9—13) comprises a second Venturi tube (13) connected, by its downstream end, to the upstream end of the first Venturi tube (14) and by its upstream end to an electro-valve (9) connected between the source (2) of gas supply, via a throttle (10), and the second Venturi tube (13) via a capacitor (11) and another throttle (9), the electro-valve (9) being connected to the output of the computer (16).

2. An apparatus according to claim 1 characterized in that the computer (16) is provided with a member (17) such as a potentiometer predetermining the threshold of the minimum current volume of gas decided by the prescriber of assisted respiration.

## Fig. 1

## Fig. 2